# EUROPEAN PATENT APPLICATION

(11) **EP 2 574 340 A2**
(43) Date of publication of application: **03.04.2013**
(21) Application number: 12197654.2
(22) Date of filing: 05.04.2007
(51) Int. Cl.: A61K 31/497, A61K 45/06, A61P 17/06, A61P 35/02, A61P 35/00

(54) **Combination comprising a pyrimidylaminobenzamide compound and a THR315LLEe kinase inhibitor**

(30) Priority: 07.04.2006 US 790438 P; 11.04.2006 US 791150 P
(62) Divisional of application: 07727881.0
(71) Applicant: Novartis AG, 4056 Basel (CH)
(72) Inventor: Manley, Paul W., 4144 Arlesheim (CH)
(74) Representative: Krauss, Jan

(57) **Abstract**

The invention provides a pharmaceutical combination: a) a pryrimidylaminobenzamide compound, and b) a Thr315lle kinase inhibitor and a method for treating or preventing a proliferative disease using such a combination.

## Description

The present invention relates to a pharmaceutical combination comprising a) a pyrimidylaminobenzamide compound and b) agents which inhibit Thr315Ile Bcr-Abl ("Thr315Ile inhibitor"), and the uses of such a combination in proliferative diseases, involving Abl tyrosine kinase activity, such as those in which Bcr-Abl or TEL-Abl fusion proteins are expressed. Such proliferative diseases include tumors, myelomas, leukemias, etc.

In spite of numerous treatment options for proliferative disease patients, there remains a need for effective and safe antiproliferative agents and a need for their preferential use in combination therapy.

It has now been found that combinations of AMN107 with agents that inhibit Thr315Ile Bcr-Abl will provide added benefit to Leukemia patients, especially patients with chronic myeloid leukemia, ("CML") who are at risk of expressing mutant forms of Bcr-Abl, or those patients in which mutant forms have already emerged. This is because AMN107 inhibits the majority of mutants forms of Bcr-Abl (32 of 33 currently evaluated), with only the Thr315Ile mutant being AMN107 resistant and consequently combinations of AMN107 with a Thr315Ile inhibitor will be of great benefit. Such drug combinations should provide enhanced efficacy in CML and Acute Lymphocytic leukemia ("ALL") patients expressing mutant Bcr-Abl and provide an improved prognosis for patients at risk of developing Bcr-Abl mutant driven disease.

### Summary of the Invention

It has now been found that a combination comprising at a) least one pyrimidylaminobenzamide compound and b) a Thr315Ile inhibitor, e.g. as defined below, has a beneficial effect on proliferative diseases, e.g. tumors, myelomas, leukemias, psoriasis, restenosis, sclerodermitis and fibrosis.

### Detailed Description of the invention

The present invention relates to the use of pyrimidylaminobenzamide compounds of formula A: 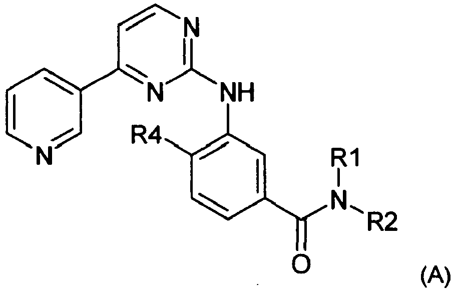 wherein
R₁ represents hydrogen, lower alkyl, lower alkoxy-lower alkyl, acyloxy-lower alkyl, carboxy-lower alkyl, lower alkoxycarbonyl-lower alkyl, or phenyl-lower alkyl;
R₂ represents hydrogen, lower alkyl, optionally substituted by one or more identical or different radicals R₃, cycloalkyl, benzcycloalkyl, heterocyclyl, an aryl group, or a mono- or bicyclic heteroaryl group comprising zero, one, two or three ring nitrogen atoms and zero or one oxygen atom and zero or one sulfur atom, which groups in each case are unsubstituted or mono- or polysubstituted;
   and R₃ represents hydroxy, lower alkoxy, acyloxy, carboxy, lower alkoxycarbonyl, carbamoyl, N-mono- or N,N-disubstituted carbamoyl, amino, mono- or disubstituted amino, cycloalkyl, heterocyclyl, an aryl group, or a mono- or bicyclic heteroaryl group comprising zero, one, two or three ring nitrogen atoms and zero or one oxygen atom and zero or one sulfur atom, which groups in each case are unsubstituted or mono- or polysubstituted;
   or wherein R₁ and R₂ together represent alkylene with four, five or six carbon atoms optionally mono- or disubstituted by lower alkyl, cycloalkyl, heterocyclyl, phenyl, hydroxy, lower alkoxy, amino, mono- or disubstituted amino, oxo, pyridyl, pyrazinyl or pyrimidinyl; benzalkylene with four or five carbon atoms; oxaalkylene with one oxygen and three or four carbon atoms; or azaalkylene with one nitrogen and three or four carbon atoms wherein nitrogen is unsubstituted or substituted by lower alkyl, phenyl-lower alkyl, lower alkoxycarbonyl-lower alkyl, carboxy-lower alkyl, carbamoyl-lower alkyl, N-mono- or N,N-disubstituted carbamoyl-lower alkyl, cycloalkyl, lower alkoxycarbonyl, carboxy, phenyl, substituted phenyl, pyridinyl, pyrimidinyl, or pyrazinyl;
R₄ represents hydrogen, lower alkyl, or halogen;
and a N-oxide or a pharmaceutically acceptable salt of such a compound for the preparation of a pharmaceutical composition for the treatment of kinase dependent diseases.

The general terms used hereinbefore and hereinafter preferably have within the context of this disclosure the following meanings, unless otherwise indicated:

The prefix "lower" denotes a radical having up to and including a maximum of 7, especially up to and including a maximum of 4 carbon atoms, the radicals in question being either linear or branched with single or multiple branching.

Where the plural form is used for compounds, salts, and the like, this is taken to mean also a single compound, salt, or the like.

Any asymmetric carbon atoms may be present in the (R)-, (S)- or (R,S)-configuration, preferably in the (R)- or (S)-configuration. The compounds may thus be present as mixtures of isomers or as pure isomers, preferably as enantiomer-pure diastereomers.

The invention relates also to possible tautomers of the compounds of formula A.

Lower alkyl is preferably alkyl with from and including 1 up to and including 7, preferably from and including 1 to and including 4, and is linear or branched; preferably, lower alkyl is butyl, such as n-butyl, sec-butyl, isobutyl, tert-butyl, propyl, such as n-propyl or isopropyl, ethyl or methyl. Preferably lower alkyl is methyl, propyl or tert-butyl.

Lower acyl is preferably formyl or lower alkylcarbonyl, in particular acetyl.

An aryl group is an aromatic radical which is bound to the molecule via a bond located at an aromatic ring carbon atom of the radical. In a preferred embodiment, aryl is an aromatic radical having 6 to 14 carbon atoms, especially phenyl, naphthyl, tetrahydronaphthyl, fluorenyl or phenanthrenyl, and is unsubstituted or substituted by one or more, preferably up to three, especially one or two substituents, especially selected from amino, mono- or disubstituted amino, halogen, lower alkyl, substituted lower alkyl, lower alkenyl, lower alkynyl, phenyl, hydroxy, etherified or esterified hydroxy, nitro, cyano, carboxy, esterified carboxy, alkanoyl, benzoyl, carbamoyl, N-mono- or N,N-disubstituted carbamoyl, amidino, guanidino, ureido, mercapto, sulfo, lower alkylthio, phenylthio, phenyl-lower alkylthio, lower alkylphenylthio, lower alkylsulfinyl, phenylsulfinyl, phenyl-lower alkylsulfinyl, lower alkylphenylsulfinyl, lower alkylsulfonyl, phenylsulfonyl, phenyl-lower alkylsulfonyl, lower alkylphenylsulfonyl, halogen-lower alkylmercapto, halogen-lower alkylsulfonyl, such as especially trifluoromethanesulfonyl, dihydroxybora (-B(OH)₂), heterocyclyl, a mono- or bicyclic heteroaryl group and lower alkylene dioxy bound at adjacent C-atoms of the ring, such as methylene dioxy. Aryl is more preferably phenyl, naphthyl or tetrahydronaphthyl, which in each case is either unsubstituted or independently substituted by one or two substituents selected from the group comprising halogen, especially fluorine, chlorine, or bromine; hydroxy; hydroxy etherified by lower alkyl, e.g. by methyl, by halogen-lower alkyl, e.g. trifluoromethyl, or by phenyl; lower alkylene dioxy bound to two adjacent C-atoms, e.g. methylenedioxy, lower alkyl, e.g. methyl or propyl; halogen-lower alkyl, e.g. trifluoromethyl; hydroxy-lower alkyl, e.g. hydroxymethyl or 2-hydroxy-2-propyl; lower alkoxy-lower alkyl; e.g. methoxymethyl or 2-methoxyethyl; lower alkoxycarbonyl-lower alkyl, e.g. methoxycarbonylmethyl; lower alkynyl, such as 1-propynyl; esterified carboxy, especially lower alkoxycarbonyl, e.g. methoxycarbonyl, n-propoxy carbonyl or iso-propoxy carbonyl; N-monosubstituted carbamoyl, in particular carbamoyl monosubstituted by lower alkyl, e.g. methyl, n-propyl or iso-propyl; amino; lower alkylamino, e.g. methylamino; di-lower alkylamino, e.g. dimethylamino or diethylamino; lower alkylene-amino, e.g. pyrrolidino or piperidino; lower oxaalkylene-amino, e.g. morpholino, lower azaalkylene-amino, e.g. piperazino, acylamino, e.g. acetylamino or benzoylamino; lower alkylsulfonyl, e.g. methylsulfonyl; sulfamoyl; or phenylsulfonyl.

A cycloalkyl group is preferably cyclopropyl, cyclopentyl, cyclohexyl or cycloheptyl, and may be unsubstituted or substituted by one or more, especially one or two, substituents selected from the group defined above as substituents for aryl, most preferably by lower alkyl, such as methyl, lower alkoxy, such as methoxy or ethoxy, or hydroxy, and further by oxo or fused to a benzo ring, such as in benzcyclopentyl or benzcyclohexyl.

Substituted alkyl is alkyl as last defined, especially lower alkyl, preferably methyl; where one or more, especially up to three, substituents may be present, primarily from the group selected from halogen, especially fluorine, amino, N-lower alkylamino, N,N-di-lower alkylamino, N-lower alkanoylamino, hydroxy, cyano, carboxy, lower alkoxycarbonyl, and phenyl-lower alkoxycarbonyl. Trifluoromethyl is especially preferred.

Mono- or disubstituted amino is especially amino substituted by one or two radicals selected independently of one another from lower alkyl, such as methyl; hydroxy-lower alkyl, such as 2-hydroxyethyl; lower alkoxy lower alkyl, such as methoxy ethyl; phenyl-lower alkyl, such as benzyl or 2-phenylethyl; lower alkanoyl, such as acetyl; benzoyl; substituted benzoyl, wherein the phenyl radical is especially substituted by one or more, preferably one or two, substituents selected from nitro, amino, halogen, N-lower alkylamino, N,N-di-lower alkylamino, hydroxy, cyano, carboxy, lower alkoxycarbonyl, lower alkanoyl, and carbamoyl; and phenyl-lower alkoxycarbonyl, wherein the phenyl radical is unsubstituted or especially substituted by one or more, preferably one or two, substituents selected from nitro, amino, halogen, N-lower alkylamino, N,N-di-lower alkylamino, hydroxy, cyano, carboxy, lower alkoxycarbonyl, lower alkanoyl, and carbamoyl; and is preferably N-lower alkylamino, such as N-methylamino, hydroxy-lower alkylamino, such as 2-hydroxyethylamino or 2-hydroxypropyl, lower alkoxy lower alkyl, such as methoxy ethyl, phenyl-lower alkylamino, such as benzylamino, N,N-di-lower alkylamino, N-phenyl-lower alkyl-N-lower alkylamino, N,N-di-lower alkylphenylamino, lower alkanoylamino, such as acetylamino, or a substituent selected from the group comprising benzoylamino and phenyl-lower alkoxycarbonylamino, wherein the phenyl radical in each case is unsubstituted or especially substituted by nitro or amino, or also by halogen, amino, N-lower alkylamino, N,N-di-lower alkylamino, hydroxy, cyano, carboxy, lower alkoxycarbonyl, lower alkanoyl, carbamoyl or aminocarbonylamino. Disubstituted amino is also lower alkylene-amino, e.g. pyrrolidino, 2-oxopyrrolidino or piperidino; lower oxaalkylene-amino, e.g. morpholino, or lower azaalkylene-amino, e.g. piperazino or N-substituted piperazino, such as N-methylpiperazino or N-methoxycarbonylpiperazino.

Halogen is especially fluorine, chlorine, bromine, or iodine, especially fluorine, chlorine, or bromine.

Etherified hydroxy is especially C₈-C₂₀alkyloxy, such as n-decyloxy, lower alkoxy (preferred), such as methoxy, ethoxy, isopropyloxy, or tert-butyloxy, phenyl-lower alkoxy, such as benzyloxy, phenyloxy, halogen-lower alkoxy, such as trifluoromethoxy, 2,2,2-trifluoroethoxy or 1,1,2,2-tetrafluoroethoxy, or lower alkoxy which is substituted by mono- or bicyclic heteroaryl comprising one or two nitrogen atoms, preferably lower alkoxy which is substituted by imidazolyl, such as 1H-imidazol-1-yl, pyrrolyl, benzimidazolyl, such as 1-benzimidazolyl, pyridyl, especially 2-, 3- or 4-pyridyl, pyrimidinyl, especially 2-pyrimidinyl, pyrazinyl, isoquinolinyl, especially 3-isoquinolinyl, quinolinyl, indolyl or thiazolyl.

Esterified hydroxy is especially lower alkanoyloxy, benzoyloxy, lower alkoxycarbonyloxy, such as tert-butoxycarbonyloxy, or phenyl-lower alkoxycarbonyloxy, such as benzyloxycarbonyloxy.

Esterified carboxy is especially lower alkoxycarbonyl, such as tert-butoxycarbonyl, isopropoxycarbonyl, methoxycarbonyl or ethoxycarbonyl, phenyl-lower alkoxycarbonyl, or phenyloxycarbonyl.

Alkanoyl is primarily alkylcarbonyl, especially lower alkanoyl, e.g. acetyl.

N-Mono- or N,N-disubstituted carbamoyl is especially substituted by one or two substituents independently selected from lower alkyl, phenyl-lower alkyl and hydroxy-lower alkyl, or lower alkylene, oxa-lower alkylene or aza-lower alkylene optionally substituted at the terminal nitrogen atom.

A mono- or bicyclic heteroaryl group comprising zero, one, two or three ring nitrogen atoms and zero or one oxygen atom and zero or one sulfur atom, which groups in each case are unsubstituted or mono- or polysubstituted, refers to a heterocyclic moiety that is unsaturated in the ring binding the heteroaryl radical to the rest of the molecule in formula I and is preferably a ring, where in the binding ring, but optionally also in any annealed ring, at least one carbon atom is replaced by a heteroatom selected from the group consisting of nitrogen, oxygen and sulfur; where the binding ring preferably has 5 to 12, more preferably 5 or 6 ring atoms; and which may be unsubstituted or substituted by one or more, especially one or two, substituents selected from the group defined above as substituents for aryl, most preferably by lower alkyl, such as methyl, lower alkoxy, such as methoxy or ethoxy, or hydroxy. Preferably the mono- or bicyclic heteroaryl group is selected from 2H-pyrrolyl, pyrrolyl, imidazolyl, benzimidazolyl, pyrazolyl, indazolyl, purinyl, pyridyl, pyrazinyl, pyrimidinyl, pyridazinyl, 4H-quinolizinyl, isoquinolyl, quinolyl, phthalazinyl, naphthyridinyl, quinoxalyl, quinazolinyl, quinnolinyl, pteridinyl, indolizinyl, 3H-indolyl, indolyl, isoindolyl, oxazolyl, isoxazolyl, thiazolyl, isothiazolyl, triazolyl, tetrazolyl, furazanyl, benzo[d]pyrazolyl, thienyl and furanyl. More preferably the mono- or bicyclic heteroaryl group is selected from the group consisting of pyrrolyl, imidazolyl, such as 1H-imidazol-1-yl, benzimidazolyl, such as 1-benzimidazolyl, indazolyl, especially 5-indazolyl, pyridyl, especially 2-, 3- or 4-pyridyl, pyrimidinyl, especially 2-pyrimidinyl, pyrazinyl, isoquinolinyl, especially 3-isoquinolinyl, quinolinyl, especially 4- or 8-quinolinyl, indolyl, especially 3-indolyl, thiazolyl, benzo[d]pyrazolyl, thienyl, and furanyl. In one preferred embodiment of the invention the pyridyl radical is substituted by hydroxy in ortho position to the nitrogen atom and hence exists at least partially in the form of the corresponding tautomer which is pyridin-(1H)2-one. In another preferred embodiment, the pyrimidinyl radical is substituted by hydroxy both in position 2 and 4 and hence exists in several tautomeric forms, e.g. as pyrimidine-(1H, 3H)2,4-dione.

Heterocyclyl is especially a five, six or seven-membered heterocyclic system with one or two heteroatoms selected from the group comprising nitrogen, oxygen, and sulfur, which may be unsaturated or wholly or partly saturated, and is unsubstituted or substituted especially by lower alkyl, such as methyl, phenyl-lower alkyl, such as benzyl, oxo, or heteroaryl, such as 2-piperazinyl; heterocyclyl is especially 2- or 3-pyrrolidinyl, 2-oxo-5-pyrrolidinyl, piperidinyl, N-benzyl-4-piperidinyl, N-lower alkyl-4-piperidinyl, N-lower alkyl-piperazinyl, morpholinyl, e.g. 2-or 3-morpholinyl, 2-oxo-1H-azepin-3-yl, 2-tetrahydrofuranyl, or 2-methyl-1,3-dioxolan-2-yl.

Salts are especially the pharmaceutically acceptable salts of compounds of formula A.

Such salts are formed, for example, as acid addition salts, preferably with organic or inorganic acids, from compounds of formula I with a basic nitrogen atom, especially the pharmaceutically acceptable salts. Suitable inorganic acids are, for example, halogen acids, such as hydrochloric acid, sulfuric acid, or phosphoric acid. Suitable organic acids are, for example, carboxylic, phosphonic, sulfonic or sulfamic acids, for example acetic acid, propionic acid, octanoic acid, decanoic acid, dodecanoic acid, glycolic acid, lactic acid, fumaric acid, succinic acid, adipic acid, pimelic acid, suberic acid, azelaic acid, malic acid, tartaric acid, citric acid, amino acids, such as glutamic acid or aspartic acid, maleic acid, hydroxymaleic acid, methylmaleic acid, cyclohexanecarboxylic acid, adamantanecarboxylic acid, benzoic acid, salicylic acid, 4-aminosalicylic acid, phthalic acid, phenylacetic acid, mandelic acid, cinnamic acid, methane- or ethane-sulfonic acid, 2-hydroxyethanesulfonic acid, ethane-1,2-disulfonic acid, benzenesulfonic acid, 2-naphthalenesulfonic acid, 1,5-naphthalene-disulfonic acid, 2-, 3- or 4-methylbenzenesulfonic acid, methylsulfuric acid, ethylsulfuric acid, dodecylsulfuric acid, N-cyclohexylsulfamic acid, N-methyl-, N-ethyl- or N-propyl-sulfamic acid, or other organic protonic acids, such as ascorbic acid.

In the presence of negatively charged radicals, such as carboxy or sulfo, salts may also be formed with bases, e.g. metal or ammonium salts, such as alkali metal or alkaline earth metal salts, for example sodium, potassium, magnesium or calcium salts, or ammonium salts with ammonia or suitable organic amines, such as tertiary monoamines, for example triethylamine or tri(2-hydroxyethyl)amine, or heterocyclic bases, for example N-ethylpiperidine or N,N'-dimethylpiperazine.

When a basic group and an acid group are present in the same molecule, a compound of formula A may also form internal salts.

For isolation or purification purposes it is also possible to use pharmaceutically unacceptable salts, for example picrates or perchlorates. For therapeutic use, only pharmaceutically acceptable salts or free compounds are employed (where applicable in the form of pharmaceutical preparations), and these are therefore preferred.

In view of the close relationship between the novel compounds in free form and those in the form of their salts, including those salts that can be used as intermediates, for example in the purification or identification of the novel compounds, any reference to the free compounds hereinbefore and hereinafter is to be understood as referring also to the corresponding salts, as appropriate and expedient.

Compounds within the scope of formula A and the process for their manufacture are disclosed in WO 04/005281 published on January 15, 2004 which is hereby incorporated into the present application by reference. A preferred compound is 4-Methyl-3-[[4-(3-pyridinyl)-2-pyrimidinyl]amino]-*N*-[5-(4-methyl-1H-imidazol-1-yl)-3-(trifluoromethyl)phenyl] benzamide. Combinations of the present invention include compounds which target the tyrosine kinase activity of Thr315Ile Bcr-Abl. Such compounds or agents will be referred to as "Thr315Ile inhibitors".

Suitable Thr315Ile inhibitors include e.g.:
a. Compounds described in WO 05/039486, WO 05/03869, WO 05/123719, WO 03/099771, WO 02/057259 and WO 04/00083 which are incorporated herein by reference. A preferred compound disclosed by WO 05/123719 is N-[3-[[1-[6-(cyclopropylamino)-4-pyrimidinyl]-1h-imadazol-2-yl]amino]-4-methylphenyl]-3-(4-methyl-1H-imidazol-1-yl)-5-(trifluoromethyl)-benzamide or a pharmaceutically acceptable salt thereof.
b. Cyclic diaryl ureas of Formula I or salts, esters, N-oxides or prodrugs thereof: 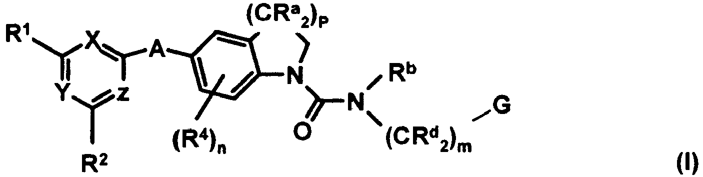 wherein
   pis1,2or3;
   m is 0, 1, 2 or 3;
   n is 0, 1, 2 or 3;
   A is CR^{c}, S, NR^{c} or O where R^{c} is H or lower alkyl;
   X, Y and Z are each independently selected fro N or C-R³ wherein at least two of X, Y and Z are N; and
   each R^{a} and R^{d} are independently selected from hydrogen and lower-alkyl;
   each R^{b} is hydrogen or lower-alkyl;
   R¹, R² and R³ are each independently selected from an organic or inorganic moiety, wherein the inorganic moiety is especially selected from halo, especially chloro, hydroxyl, etherified and esterified hydroxyl, cyano, azo (N=N=N) and nitro; and
   where the organic moiety is substituted or unsubstituted and may be attached via a linker, -L¹-, the organic moiety being especially selected from hydrogen; lower alkyl, especially linear or branched C₁-C₆ alkyl, lower alkenyl or lower alkynyl, optionally substituted with one or more substituents and/or interrupted by one or more heteroatoms; lower alkoxy, especially methoxy or ethoxy; lower-alkanoyl; aroyl; heteroaroyl; carboxy; carboxamido which unsubstituted or substituted by linear or branched C₁-C₆ alkyl; amino; a cyclic group, for example cycloalkyl, e.g. cyclohexyl, phenyl, pyrrole, imidazole, pyrazole, isoxazole, oxazole, thiazole, pyridazine, pyrimidine, pyrazine, pyridyl, indole, isoindole, indazole, purine, indolizidine, quinoline, isoquinoline, quinazoline, pteridine, quinolizidine, piperidyl, piperazinyl, pyrollidine, morpholinyl and thiomorpholinyl; or
   mono- or di- substituted amino, the amino optionally being substituted by a hydrocarbyl moiety, the hydrocarbyl moiety being, for example, selected from lower alkyl, especially linear or branched C₁ - C₆ alkyl, cycloalkyl, especially cyclohexyl, carboxy, lower alkanoyl, especially acetyl, lower alkoxy carbonyl, lower alkyl sulfonyl, aroyl, such as benzoyl or nicotinoyl, a carbocyclic group, for example phenyl, a heterocyclic group and heterocyclyl carbonyl; where the hydrocarbyl moiety is substituted or unsubstituted;
   and -L¹- having 1, 2, 3 or 4 in-chain atoms (e.g. selected from C, N, O and S) and optionally being selected from C₁, C₂, C₃ or C₄ alkyl; such an alkyl group optionally being interrupted and/or terminated by an -O- or -NH- linkage; O; N; or S; and wherein -L¹- can also be carbonyl;
   wherein there is at least one of R¹, R² or R³ which is not hydrogen; and
   when Z is C-R³, either R¹ is not H or R² is not Cl, or both;
   wherein, when X represent CR³, R³ and R¹ together with the carbon atoms to which they are attached form a five- or six-membered unsaturated ring containing at least one nitrogen atom;
   R⁴ is selected from an organic or inorganic moiety, for example, R⁴ is selected from halogen, lower alkyl, halo-lower alkyl, carboxy, lower alkoxycarbonyl, hydroxy, etherified or esterified hydroxy, lower alkoxy, phenyl, substituted phenyl, for example phenyl-loweralkoxy, lower alkanoyloxy, lower alkanoyl, amino, mono- or di- substituted amino, amidino, ureido, mercapto, N-hydroxy-amidino, guanidino, amidino-lower alkyl, sulfo, sulfamoyl, carbamoyl, cyano, cyano-lower alkyl and nitro.
   R⁴ is commonly selected from hydroxy, lower alkyl or halo (notably F or Cl). n is preferably zero or 1.
   Cyclic diaryl ureas within the scope of formula I and the process for their manufacture are disclosed in PCT/EP2005/010408 published on April 6th, 2006 under the International Publication number WO2006/034833 which is hereby incorporated into the present application by reference. A preferred compound is [6-[[1-[[4-[(4-Methyl-1-piperazinyl)methyl]-3-(trifluoromethyl)phenylamino]carbonyl]-1H-indol-5-yl]oxy]-4-pyrimidinyl]acetamide.
c. Compounds of the formula I: 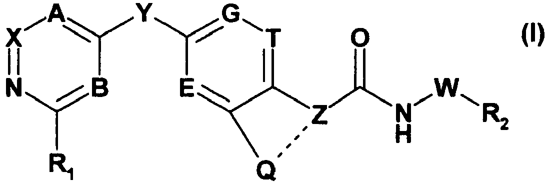 wherein
   R₁ is H; halo; -C₀-C₇O-R₃; -C₀-C₇-NR₄R₅; or -C(=O)-R₆;
   R₂ is substituted C₃-C₈-cycloalkyl; substituted aryl; or substituted heterocyclyl;
   R₃ is H or unsubstituted or substituted lower alkyl;
   R₄ and R₅ are independently selected from the group consisting of H; unsubstituted or substituted lower alkyl; lower alkyl-carbonyl, wherein the lower alkyl moiety is optionally substituted; and lower alkoxy-carbonyl, wherein the lower alkyl moiety is optionally substituted;
   R₆ is H; unsubstituted or substituted lower alkyl; lower alkoxy, wherein the lower alkyl moiety is optionally substituted; or unsubstituted, mono- or di-substituted amino;
   A, B and X are independently selected from =C(R₇)- or N;
   E, G and T are independently selected from =C(R₈)- or N;
   R₇ and R₈ are independently selected from the group consisting of H, halo and unsubstituted or substituted lower alkyl;
   Y is -O-, -S-, -S(O)-, -S(O)₂-, -CH₂- or -CH₂-CH₂- ;
   Z is CH or N and Q is C₁-C₄-alkylene or C₂-C₄-alkenylene, wherein C₁-C₄-alkylene or C₂-C₄-alkenylene optionally may be substituted and wherein one or more of the carbon atoms of said C₁-C₄-alkylene or C₂-C₄-alkenylene chain optionally may be replaced by a heteroatom independently selected from nitrogen, oxygen and sulfur; and the bond between Q and Z characterized by a dotted line is a single bond; with the proviso that if Z is N, Q is not unsubstituted unbranched C₁-C₄-alkylene;
   or
   Z is C and Q is as defined above wherein the bond between Q and Z characterized by a dotted line is a double bond; and
   W is either not present or C₁-C₃-alkylene;
   or a tautomer thereof, or a salt thereof.
   Compounds within the scope of formula I and the process for their manufacture are disclosed in PCT/IB2005/004030, published on June 8th, 2006 under the International Publication number WO2006/059234, which is hereby incorporated into the present application by reference. A preferred compound is: 6-(2-Amino-pyrimidin-4-yloxy)-naphthalene-1-carboxylic acid (3-trifluoromethyl-phenyl)-amide.
d. Additional compounds are compound AP24534 from ARIAD Pharmaceuticals, Inc. and XL228 from Exelixis, Inc.

Comprised are likewise the pharmaceutically acceptable salts thereof, the corresponding racemates, diastereoisomers, enantiomers, tautomers, as well as the corresponding crystal modifications of above disclosed compounds where present, e.g. solvates, hydrates and polymorphs, which are disclosed therein. The compounds used as active ingredients in the combinations of the invention can be prepared and administered as described in the cited documents, respectively. Also within the scope of this invention is the combination of more than two separate active ingredients as set forth above, i.e., a pharmaceutical combination within the scope of this invention could include three active ingredients or more.

In accordance with the particular findings of the present invention, there is provided
1. A pharmaceutical combination comprising:
   a) a pyrimidylaminobenzamide compound of formula A; and
   b) at least one Thr315Ileinhibitor.
2. A method for treating or preventing proliferative disease in a subject in need thereof, comprising co-administration to said subject, e.g., concomitantly or in sequence, of a therapeutically effective amount of a pyrimidylaminobenzamide compound of formula A and a Thr315Ile inhibitor, e.g., as disclosed above.
   Examples of proliferative diseases include e.g. tumors, leukemias psoriasis, restenosis, sclerodermitis and fibrosis. Especially preferred is the treatment of leukemias such as CML and leukemias resistant to imatinib (Gleevec® or STI571).
3. A pharmaceutical combination as defined under 1) above, e.g. for use in a method as defined under 2) above.
4. A pharmaceutical combination as defined under 1) above for use in the preparation of a medicament for use in a method as defined under 2) above.
5. A pharmaceutical combination as defined under 1 wherein agent a) is 4-Methyl-3-[[4-(3-pyridinyl)-2-pyrimidinyl]amino]-*N*-[5-(4-methyl-1H-imidazol-1-yl)-3 (trifluoromethyl)phenyl] benzamide or a pharmaceutically acceptable salt thereof.
6. A method for treating leukemia comprising administering a combination of a Thr315Ile inhibitor and a pyrimidylaminobenzamide compound of formula A.
7. A method for treating leukemia comprising administering a combination of a Thr315Ile inhibitor and 4-Methyl-3-[[4-(3-pyridinyl)-2-pyrimidinyl]amino]-N-[5-(4-methyl-1H-imidazol-1-yl)-3-(trifluoromethyl)phenyl] benzamide.
8. A method as defined under 7 where the Thr315Ile inhibitor is selected from 6-(2-Amino-pyrimidin-4-yloxy)-naphthalene-1-carboxylic acid (3-trifluoromethyl-phenyl)-amide; [6-[[1-[[4-[(4-Methyl-1-piperazinyl)methyl]-3 (trifluoromethyl)phenylamino] carbonyl]-1H-indol-5-yl]oxy]-4-pyrimidinyl]acetamide; N-[3-[[1-[6-(cyclopropylamino)-4-pyrimidinyl]-1h-imadazol-2-yl]amino]-4-methylphenyl]-3-(4-methyl-1H-imidazol-1-yl)-5-(trifluoromethyl)-benzamide, XL228, AP24534 and combinations thereof.
   Utility of the combination of the invention in a method as hereinabove specified, may be demonstrated in animal test methods as well as in clinic, for example in accordance with the methods hereinafter described.

### A. Combined Treatment

Suitable clinical studies are, for example, open label, dose escalation studies in patients with proliferative diseases. Such studies prove in particular the synergism of the active ingredients of the combination of the invention. The beneficial effects on psoriasis or multiple sclerosis can be determined directly through the results of these studies which are known as such to a person skilled in the art. Such studies are, in particular, suitable to compare the effects of a monotherapy using the active ingredients and a combination of the invention. Preferably, the dose of agent (a) is escalated until the Maximum Tolerated Dosage is reached, and agent (b) is administered with a fixed dose. Alternatively, the agent (a) is administered in a fixed dose and the dose of agent (b) is escalated. Each patient receives doses of the agent (a) either daily or intermittent. The efficacy of the treatment can be determined in such studies, e.g., after 12, 18 or 24 weeks by evaluation of symptom scores every 6 weeks.

The administration of a pharmaceutical combination of the invention results not only in a beneficial effect, e.g. a synergistic therapeutic effect, e.g. with regard to alleviating, delaying progression of or inhibiting the symptoms, but also in further surprising beneficial effects, e.g. fewer side-effects, an improved quality of life or a decreased morbidity, compared with a monotherapy applying only one of the pharmaceutically active ingredients used in the combination of the invention.

A further benefit is that lower doses of the active ingredients of the combination of the invention can be used, for example, that the dosages need not only often be smaller but are also applied less frequently, which may diminish the incidence or severity of side-effects. This is in accordance with the desires and requirements of the patients to be treated.

The terms "co-administration" or "combined administration" or the like as utilized herein are meant to encompass administration of the selected therapeutic agents to a single patient, and are intended to include treatment regimens in which the agents are not necessarily administered by the same route of administration or at the same time.

It is one objective of this invention to provide a pharmaceutical composition comprising a quantity, which is jointly therapeutically effective at targeting or preventing proliferative diseases a combination of the invention. In this composition, agent (a) and agent (b) may be administered together, one after the other or separately in one combined unit dosage form or in two separate unit dosage forms. The unit dosage form may also be a fixed combination.

The pharmaceutical compositions for separate administration of agent (a) and agent (b) or for the administration in a fixed combination, i.e. a single galenical composition comprising at least two combination partners (a) and (b), according to the invention may be prepared in a manner known per se and are those suitable for enteral, such as oral or rectal, and parenteral administration to mammals (warm-blooded animals), including humans, comprising a therapeutically effective amount of at least one pharmacologically active combination partner alone, e.g. as indicated above, or in combination with one or more pharmaceutically acceptable carriers or diluents, especially suitable for enteral or parenteral application.

Suitable pharmaceutical compositions contain, for example, from about 0.1 % to about 99.9%, preferably from about 1 % to about 60 %, of the active ingredient(s). Pharmaceutical preparations for the combination therapy for enteral or parenteral administration are, for example, those in unit dosage forms, such as sugar-coated tablets, tablets, capsules or suppositories, or ampoules. If not indicated otherwise, these are prepared in a manner known per se, for example by means of conventional mixing, granulating, sugar-coating, dissolving or lyophilizing processes. It will be appreciated that the unit content of a combination partner contained in an individual dose of each dosage form need not in itself constitute an effective amount since the necessary effective amount can be reached by administration of a plurality of dosage units.

In particular, a therapeutically effective amount of each of the combination partner of the combination of the invention may be administered simultaneously or sequentially and in any order, and the components may be administered separately or as a fixed combination. For example, the method of preventing or treating proliferative diseases according to the invention may comprise (i) administration of the first agent (a) in free or pharmaceutically acceptable salt form and (ii) administration of an agent (b) in free or pharmaceutically acceptable salt form, simultaneously or sequentially in any order, in jointly therapeutically effective amounts, preferably in synergistically effective amounts, e.g. in daily or intermittently dosages corresponding to the amounts described herein. The individual combination partners of the combination of the invention may be administered separately at different times during the course of therapy or concurrently in divided or single combination forms. Furthermore, the term administering also encompasses the use of a pro-drug of a combination partner that convert *in vivo* to the combination partner as such. The instant invention is therefore to be understood as embracing all such regimens of simultaneous or alternating treatment and the term "administering" is to be interpreted accordingly.

The effective dosage of each of the combination partners employed in the combination of the invention may vary depending on the particular compound or pharmaceutical composition employed, the mode of administration, the condition being treated, the severity of the condition being treated. Thus, the dosage regimen of the combination of the invention is selected in accordance with a variety of factors including the route of administration and the renal and hepatic function of the patient. A clinician or physician of ordinary skill can readily determine and prescribe the effective amount of the single active ingredients required to alleviate, counter or arrest the progress of the condition. Optimal precision in achieving concentration of the active ingredients within the range that yields efficacy without toxicity requires a regimen based on the kinetics of the active ingredients' availability to target sites.

Daily dosages for agent (a) or (b) or will, of course, vary depending on a variety of factors, for example the compound chosen, the particular condition to be treated and the desired effect. In general, however, satisfactory results are achieved on administration of agent (a) at daily dosage rates of the order of ca. 0.03 to 5 mg/kg per day, particularly 0.1 to 5 mg/kg per day, e.g. 0.1 to 2.5 mg/kg per day, as a single dose or in divided doses. Agent (a) and agent (b) may be administered by any conventional route, in particular enterally, e.g. orally, e.g. in the form of tablets, capsules, drink solutions or parenterally, e.g. in the form of injectable solutions or suspensions. Suitable unit dosage forms for oral administration comprise from ca. 0.02 to 50 mg active ingredient, usually 0.1 to 30 mg, e.g. agent (a) or (b), together with one or more pharmaceutically acceptable diluents or carriers therefore.

Agent (b) may be administered to a human in a daily dosage range of 0.5 to 1000 mg. Suitable unit dosage forms for oral administration comprise from ca. 0.1 to 500 mg active ingredient, together with one or more pharmaceutically acceptable diluents or carriers therefore.

The administration of a pharmaceutical combination of the invention results not only in a beneficial effect, e.g. a synergistic therapeutic effect, e.g. with regard to inhibiting the unregulated proliferation of haematological stem cells or slowing down the progression of leukemias, such as CML or AML, or the growth of tumors, but also in further surprising beneficial effects, e.g. less side-effects, an improved quality of life or a decreased morbidity, compared to a monotherapy applying only one of the pharmaceutically active ingredients used in the combination of the invention.

A further benefit is that lower doses of the active ingredients of the combination of the invention can be used, for example, that the dosages need not only often be smaller but are also applied less frequently, or can be used in order to diminish the incidence of side-effects. This is in accordance with the desires and requirements of the patients to be treated.

### B. Diseases to be treated

The term "proliferative disease" includes but is not restricted to tumors, psoriasis, restenosis, sclerodermitis and fibrosis.

The term "hematological malignancy", refers in particular to leukemias, especially those expressing Bcr-Abl, c-Kit or Fit-3, and includes, but is not limited to, chronic myelogenous leukemia (CML) and acute lymphocyte leukemia (ALL), especially the Philadelphia chromosome positive acute lymphocyte leukemia (Ph+ALL) as well as ST1571-resistant (or imatinib-resistant) leukemias and cells expressing imatinib resistant Bcr-abl mutations such as Bcr-Abl^{T3151}.

The term "a solid tumor disease" especially means ovarian cancer, breast cancer, cancer of the colon and generally the gastrointestinal tract, cervix cancer, lung cancer, e.g. small-cell lung cancer and non-small-cell lung cancer, head and neck cancer, bladder cancer, cancer of the prostate or Kaposi's sarcoma.

The combinations according to the invention, that inhibit the protein kinase activities mentioned, especially tyrosine protein kinases mentioned above and below, can therefore be used in the treatment of protein kinase dependent diseases. Protein kinase dependent diseases are especially proliferative diseases, preferably benign or especially malignant tumours (for example carcinoma of the kidneys, liver, adrenal glands, bladder, breast, stomach, ovaries, colon, rectum, prostate, pancreas, lungs, vagina or thyroid, sarcoma, glioblastomas and numerous tumours of the neck and head, as well as leukemias). They are able to bring about the regression of tumours and to prevent the formation of tumour metastases and the growth of (also micro)metastases. In addition they can be used in epidermal hyperproliferation (e.g. psoriasis), in prostate hyperplasia, and in the treatment of neoplasias, especially of epithelial character, for example mammary carcinoma. It is also possible to use the combinations of the present invention in the treatment of diseases of the immune system insofar as several or, especially, individual tyrosine protein kinases are involved; furthermore, the combinations of the present invention can be used also in the treatment of diseases of the central or peripheral nervous system where signal transmission by at least one tyrosine protein kinase, especially selected from those mentioned specifically, is involved.

In chronic myelogeous leukemia (CML), a reciprocally balanced chromosomal translocation in hematopoietic stem cells (HSCs) produces the Bcr-Abl hybrid gene. The latter encodes the oncogenic Bcr-Abl fusion protein. Whereas ABL encodes a tightly regulated protein tyrosine kinase, which plays a fundamental role in regulating cell proliferation, adherence and apoptosis, the Bcr-Abl fusion gene encodes as constitutively activated kinase, which transforms HSCs to produce a phenotype exhibiting deregulated clonal proliferation, reduced capacity to adhere to the bone marrow stroma and a reduces apoptotic response to mutagenic stimuli, which enable it to accumulate progressively more malignant transformations. The resulting granulocytes fail to develop into mature lymphocytes and are released into the circulation, leading to a deficiency in the mature cells and increased susceptibility to infection. ATP-competitive inhibitors of Bcr-Abl have been described which prevent the kinase from activating mitogenic and anti-apoptotic pathways (e.g. P-3 kinase and STAT5), leading to the death of the Bcr-Abl phenotype cells and thereby providing an effective therapy against CML. The combinations of the present invention are thus especially appropriate for the therapy of diseases related to its overexpression, especially leukemias, such as leukemias, e.g. CML or ALL.

## Claims

1. A pharmaceutical combination comprising:
a) a pyrimidylaminobenzamide compound of formula A, and
b) at least one Thr315Ile inhibitor.

2. A method for treating or preventing a proliferative disease in a subject in need thereof, comprising co-administration to said subject, e.g. concomitantly or in sequence, of a therapeutically effective amount of at least one Thr315Ile inhibitor and a pyrimidylaminobenzamide compound of formula A.

3. A pharmaceutical combination according to claim 1 for use in a method according to claim 2.

4. A pharmaceutical combination according to claim 1 for use in the preparation of a medicament for use in a method according to claim 2.

5. A pharmaceutical combination according to claim 1 wherein agent a) is 4-Methyl-3-[[4-(3-pyridinyl)-2-pyrimidinyl]amino]-*N*-[5-(4-methyl-1H-imidazol-1-yl)-3-(trifluoromethyl)phenyl] benzamide.

6. A method for treating leukemia comprising administering a combination of a Thr315Ile inhibitor and a pyrimidylaminobenzamide compound of formula A.

7. A method for treating leukemia comprising administering a combination of a Thr315Ile inhibitor and 4-Methyl-3-[[4-(3-pyridinyl)-2-pyrimidinyl]aminol-*N*-[5-(4-methyl-1H-imidazol-1-yl)-3-(trifluoromethyl)phenyl] benzamide.

8. A method according to claim 7 where the Thr315Ile inhibitor is selected from 6-(2-Amino-pyrimidin-4-yloxy)-naphthalene-1-carboxylic acid (3-trifluoromethyl-phenyl)-amide; [6-[[1-[[4-[(4-Methyl-1-piperazinyl)methyl]-3-(trifluoromethyl)phenylamino]carbonyl]-1H-indol-5-yl]oxy]-4-pyrimidinyl]acetamide; N-[3-[[1-[6-(cyclopropylamino)-4-pyrimidinyl]-1h-imadazol-2-yl]amino]-4-methylphenyl]-3-(4-methyl-1H-imidazol-1-yl)-5-(trifluoromethyl)-benzamide, XL228, AP24534 and combinations thereof.
